# FASCICULE DE BREVET EUROPEEN

(11) **EP 4 499 800 B1**
(45) Date de publication et mention de la délivrance du brevet: **22.07.2026**
(21) Numéro de dépôt: 23714807.7
(22) Date de dépôt: 15.03.2023
(51) Int. Cl.: C12M 1/12, C12M 1/34

(54) **DISPOSITIF HUMIDIFICATEUR POUR UN DISPOSITIF DE CULTURE CELLULAIRE**
BEFEUCHTUNGSVORRICHTUNG FÜR EINE ZELLKULTURVORRICHTUNG
HUMIDIFYING DEVICE FOR A CELL CULTURE DEVICE

(30) Priorité: 31.03.2022 FR 2202942
(43) Date de publication de la demande: 05.02.2025
(73) Titulaire: Netri, 69007 Lyon (FR)
(72) Inventeur: ROUX, Serge, Cyril, 69008 LYON (FR); LARRAMENDY, Florian, Arnaud, Paul, 69390 DARDILLY (FR)
(74) Mandataire: Germain Maureau
(86) Numéro de dépôt international: PCT/FR2023/050350
(87) Numéro de publication internationale: WO 2023/187280

(56) Documents cités:
- EP-A1- 3 878 937
- WO-A1-2020/198326
- US-A1- 2014 113 360
- US-B1- 7 208 125

## Description

L'invention concerne le domaine des dispositifs de culture cellulaire et plus précisément un dispositif humidificateur d'un gaz pour un dispositif de culture cellulaire, ainsi qu'un kit.

On entend par dispositif de culture cellulaire, un dispositif conçu pour cultiver des cellules au sein d'au moins un puit de culture qui est utilisé comme une petite éprouvette. Ledit puit pouvant être fixe par rapport au dispositif de culture cellulaire ou détachable. Ainsi, un dispositif de culture cellulaire regroupe par exemple une plaque de culture formée d'un seul tenant et munie d'au moins un puit de culture ainsi qu'une boîte de culture dans laquelle la plaque de culture peut être insérée de manière détachable. Le puit de culture peut être par exemple un puit microfluidique et peut être relié à une puce microfluidique.

Les plaques de culture munies d'au moins un puit de culture sont devenues des outils standards dans la recherche et l'analyse clinique des tests de diagnostic des laboratoires. Une plaque de culture est habituellement appelée plaque « microtitre », et peut généralement contenir 6, 24, 96, 384, voire 1536 puits de culture, généralement disposés selon une matrice rectangulaire de format 2 :3, autrement dit dont le rapport largeur/longueur est égale à 2/3. Diverses caractéristiques, notamment les dimensions des puits de culture (diamètre, espacement, profondeur), ainsi que les dimensions et la rigidité de ces plaques ont été normalisées et standardisées par l'American National Standards Institute, à l'initiative de l'association SBS (Society for Biomolecular Screening). Les coordonnées géométriques des centres des puits sont standardisés par style, le style d'une plaque étant déterminé par le nombre de puits de la plaque. Les plaques de culture sont généralement réalisées polystyrène, également désigné par l'abréviation PS, qui est un thermoplastique transparent, biocompatible, rigide, peu cher, et facile à mouler. De manière également connue, les plaques de culture, et plus spécifiquement les plaques de culture pour des applications microfluidiques peuvent être réalisées en polydiméthylsiloxane ou diméthicole, également désigné par l'abréviation PDMS, qui est un élastomère transparent, biocompatible, déformable, très facile à mouler et qui présente des propriétés de perméabilité à l'oxygène intéressantes pour les applications microfluidiques.

Il existe des boîtes de culture configurées pour contenir au moins une plaque de culture comme par exemple celle décrite dans le document de brevet US7208125. Cette boite de culture comprend un dispositif humidificateur pour minimiser une évaporation d'un liquide des puits de culture, c'est-à-dire pour maintenir une bonne humidification de l'air présent dans la boîte de culture. Le dispositif humidificateur comprend un évidement dans lequel un liquide humidificateur peut être introduit. L'évidement est muni d'une pluralité de corps positionnés de part et d'autre d'un centre de l'évidement, ou espacés les uns par rapport aux autres d'un même côté de l'évidement. Les corps permettent notamment de minimiser un mouvement du liquide dans l'évidement, également appelé ballotement du liquide, lors d'un déplacement de la boîte de culture.

Plus précisément, le ballotement désigne des petits mouvements d'un liquide contenu dans un réservoir soumis à un mouvement accéléré et présentant une surface libre. Le ballotement inclut des mouvements dans le plan de la surface libre du liquide ainsi que des mouvements de rotation.

Ce dispositif humidificateur ne donne pas entièrement satisfaction en ce que lors de certain déplacement de la boîte de culture, du liquide peut sortir de l'évidement et se déverser dans les puits de culture.

L'invention a pour but de remédier à tout ou partie des inconvénients précités en proposant un dispositif humidificateur pour un dispositif de culture cellulaire amélioré.

L'invention a pour objet un dispositif humidificateur d'un gaz pour un dispositif de culture cellulaire, ledit dispositif humidificateur comprenant au moins un évidement configuré pour recevoir un liquide humidificateur, ledit évidement étant muni d'au moins un corps séparant une longueur dudit évidement de sorte à former un premier réservoir et un deuxième réservoir reliés l'un à l'autre par un canal, caractérisé en ce que l'au moins un corps est positionné dans l'évidement de sorte que :
- Une dimension du canal selon une largeur de l'évidement est inférieure ou égale à la moitié de la largeur de l'évidement ;
- Une dimension d'au moins un des réservoirs selon la longueur de l'évidement est inférieure ou égale à la largeur de l'évidement ;
- La dimension du réservoir selon la longueur de l'évidement est supérieure ou égale à la dimension du canal selon une largeur de l'évidement.

Le dispositif humidificateur permet une évaporation du liquide humidificateur dans le gaz du dispositif de culture cellulaire.

Préférentiellement, le liquide humidificateur comprend au moins 90% d'eau, préférence 99% d'eau, et par exemple 100% d'eau.

Le gaz entourant le dispositif de culture cellulaire contient préférentiellement de la vapeur d'eau et de l'oxygène, par exemple le gaz est de l'air avec un taux d'humidité supérieur à 80%.

Le dispositif humidificateur comprend au moins un évidement permettant de recevoir le liquide humidificateur. Préférentiellement, l'évidement n'est pas fermé de sorte à laisser une surface libre au liquide humidificateur et ainsi favoriser l'évaporation de ce dernier. L'évidement comprend donc au moins un fond, et une paroi latérale. Le fond s'étend dans un plan parallèle à un plan d'élongation du dispositif humidificateur. La paroi latérale s'étend avec un angle par rapport au plan d'élongation du dispositif humidificateur. Selon un mode de réalisation, l'angle est compris entre [30°, 150°], par exemple [60°, 120°] et préférentiellement 90°.

Selon l'invention, l'évidement comprend au moins un corps qui est positionné de sorte à séparer une longueur dudit évidement en un premier réservoir et un deuxième réservoir reliés entre eux par un canal.

On entend par la longueur de l'évidement, la plus grande dimension de l'évidement prise dans le plan d'élongation du dispositif humidificateur.

On entend par la largeur de l'évidement, la dimension de l'évidement prise dans le plan d'élongation du dispositif humidificateur et perpendiculaire à la longueur de l'évidement.

Dans le cas d'un évidement comprenant une pluralité de corps, un réservoir est compris entre deux canaux ou entre un canal et la paroi latérale de l'évidement. Ainsi, deux corps successifs définissent un réservoir en commun.

Le canal permet un passage du liquide humidificateur du premier réservoir vers le deuxième réservoir de sorte que l'ensemble de l'évidement peut être rempli en introduisant le liquide humidificateur dans un unique réservoir. Autrement dit, le liquide humidificateur s'écoule du premier réservoir vers le deuxième réservoir à travers le canal. Ainsi, un remplissage, ou un vidage, de l'évidement est rapide et facile car il peut être réalisé en versant le liquide humidificateur dans ou par un seul réservoir. Le canal présente une taille suffisante par rapport aux propriétés physiques du liquide, comme par exemple la tension superficielle, la viscosité cinématique, la densité, la mouillabilité du liquide humidificateur, pour permettre un écoulement du liquide du premier réservoir vers le deuxième réservoir.

Selon un mode de réalisation, le liquide humidificateur qui est versé dans le premier réservoir s'écoule vers le deuxième réservoir à travers le canal lorsque ledit premier réservoir est au moins en partie rempli. Autrement dit, le canal par ces dimensions bloque le passage du liquide tant que le premier réservoir ne présente pas un certain niveau de remplissage. Par exemple, le canal peut bloquer le passage du liquide tant que le premier réservoir n'est pas rempli au moins au 3/4. Ainsi, le dispositif humidificateur est rempli réservoir par réservoir.

Selon un mode de réalisation, au moins une partie de l'évidement comprend un traitement de surface permettant de modifier la mouillabilité et/ou une cinématique d'écoulement entre le liquide humidificateur et un matériau de l'évidement.

Par exemple, le traitement de surface est un traitement plasma, ou un traitement chimique qui vient altérer en surface la mouillabilité, c'est-à-dire rendre plus hydrophile ou plus hydrophobe, la surface latérale de l'évidement.

Enfin, l'évidement et le corps du dispositif humidificateur sont dimensionnés de sorte que le liquide humidificateur placé dans l'évidement soit retenu dans celui-ci lorsque le dispositif humidificateur est soumis à des déplacements, c'est-à-dire des ballotements du liquide humidificateur.

Notamment, afin de limiter le ballotement du liquide humidificateur, le corps est positionné dans l'évidement de sorte à respecter les trois critères de dimensionnement ci-dessous :
- (1) une dimension du canal selon une largeur de l'évidement est inférieure ou égale à la moitié de la largeur de l'évidement ;
- (2) une dimension d'au moins un des réservoirs selon la longueur de l'évidement est inférieure ou égale à la largeur de l'évidement ;
- (3) la dimension du réservoir selon la longueur de l'évidement est supérieure ou égale à la dimension du canal selon une largeur de l'évidement.

Le critère (1) impose que le canal forme une constriction de l'évidement. Ainsi, le canal augmente des pertes de charges du mouvement du liquide humidificateur et donc ralenti ce mouvement. Le canal permet de former des réservoirs d'un volume inférieur à celui de l'évidement tout en conservant l'avantage d'un grand volume total de l'évidement qui est facilement rempli via un seul réservoir.

Le critère (2) limite la quantité de liquide humidificateur présent dans chaque réservoir en limitant le volume dudit réservoir. En effet, les dimensions du réservoir selon la largeur et la longueur de l'évidement est au plus égale à la largeur de l'évidement. Ainsi, le volume maximum du réservoir, et donc le volume de liquide humidificateur, est limité par une surface carrée de côté égale à la largeur de l'évidement.

Limiter le volume de liquide humidificateur permet d'une part de limiter la masse de liquide humidificateur déplacée dans chaque réservoir lors d'un ballotements, et d'autre part d'augmenter un rapport entre un niveau de frottement entre le liquide humidificateur et la paroi latérale de l'évidement, et le volume du liquide humidificateur. Ainsi, il faut plus d'énergie pour mettre en mouvement le liquide humidificateur dans le réservoir que dans l'évidement sans corps, et le liquide humidificateur acquière moins d'énergie cinétique dans un volume restreint, ce qui diminue les mouvements du liquide humidificateur.

Enfin, le critère (3) impose un volume minimum du réservoir. En effet, une dimension du réservoir selon la longueur de l'évidement est supérieure ou égale à la dimension du canal selon la largeur de l'évidement. Un volume minimum du réservoir est nécessaire afin qu'il ne constitue pas en-lui-même un canal et qu'il permette d'assurer une bonne humidification du dispositif de culture cellulaire.

L'invention peut également présenter une ou plusieurs des caractéristiques suivantes prises seules ou en combinaison.

Selon un mode de réalisation, le dispositif humidificateur est configuré pour être positionné sur un bord du dispositif de culture cellulaire.

Selon un mode de réalisation, la largeur de l'évidement est comprise entre 2 et 10mm, de préférence entre 2 et 6mm, par exemple 5mm.

Selon un mode de réalisation, la dimension du canal selon la largeur de l'évidement est comprise entre 0,5mm et 5mm, et de préférence entre 0,9mm et 2,9mm.

En effet, avec ces dimensions, le canal permet de diminuer de manière importante les mouvements du liquide humidificateur.

Selon un mode de réalisation, l'évidement est réalisé dans un matériaux choisi parmi : un acide polylactique, un polystyrène et un polydiméthylsiloxane ou diméthicole.

Selon un mode de réalisation, l'évidement comprend une pluralité de corps positionnés régulièrement et/ou alternativement de part et d'autre d'un centre de l'évidement.

On entend par des corps positionnés régulièrement, qu'une distance entre les corps pris selon un axe, comme par exemple la longueur de l'évidement est constante.

On entend par des corps positionnés alternativement de part et d'autre d'un centre de l'évidement, que deux corps successifs sont positionnés d'un côté et de l'autre côté du centre de l'évidement, qui est par exemple pris selon la longueur de l'évidement.

Selon un mode de réalisation, les corps sont positionnés d'un même côté de l'évidement. Selon un mode de réalisation, l'évidement comprend une pluralité de corps définissant un chemin continu formant une sinuosité.

On entend par chemin continu, une succession de réservoirs reliés par des canaux. Autrement dit, un chemin est continu lorsqu'un liquide versé à une première extrémité du chemin peut naturellement atteindre une deuxième extrémité du chemin sans être physiquement arrêté par une barrière physique.

Selon un mode de réalisation, le corps comprend une surface de freinage configurée pour être au contact du liquide humidificateur, et une surface de retenue s'étendant dans un plan formant un angle avec un plan dans lequel s'étend la surface de freinage, ledit angle étant compris dans une première gamme de valeur, la surface de freinage étant reliée à la surface de retenue par une surface courbe présentant un rayon de courbure inférieur ou égal à 0.5mm.

La surface de freinage du corps relie le fond de l'évidement à la surface de retenue du corps. La surface de freinage s'étend avec un angle par rapport au plan dans lequel s'étend le dispositif humidificateur, l'angle étant compris entre [20° ,160°], par exemple [60° ,120°], et préférentiellement entre [89° et 92°]. Dans ce dernier cas, la surface de freinage correspond à une hauteur du corps.

La surface de retenue s'étend dans un plan formant un angle compris dans une première gamme de valeur avec un plan dans lequel s'étend la surface de freinage. La première gamme de valeur est comprise dans l'intervalle [20°, 160°], par exemple [60°, 120°], et de préférence [90°]. Dans ce dernier cas, la surface de retenue forme un angle droit avec la surface de freinage.

Selon un mode de réalisation, lorsque la surface de freinage s'étend perpendiculairement par rapport au fond de l'évidement, la surface de retenue s'étend perpendiculairement à la surface de freinage. Autrement dit, la surface de retenue s'étend dans le plan d'élongation du dispositif humidificateur.

La surface de freinage est reliée à la surface de retenue par une surface courbe présentant un rayon de courbure inférieur à 0.5mm, par exemple inférieur à 0,2mm, et de préférence 0mm.

Le rayon de courbure de la surface courbe fait varier une tension superficielle du liquide humidificateur au contact de la surface courbe. Ainsi, la surface courbe permet de favoriser une retenue du liquide humidificateur dans le réservoir. La surface courbe diminue le mouvement du liquide humidificateur selon un plan perpendiculaire au plan d'élongation du dispositif humidificateur. La géométrie du corps permet de diminuer le risque que le liquide humidificateur sorte du réservoir pour inonder un puit du dispositif de culture cellulaire.

Selon un mode de réalisation, l'au moins un corps présente une dimension selon une hauteur de l'évidement comprise entre 1mm et 15mm.

On entend par hauteur de l'évidement, la dimension de l'évidement prise dans un plan perpendiculaire au plan d'élongation du dispositif humidificateur. La hauteur de l'évidement est perpendiculaire à la longueur et à la largeur de l'évidement.

Selon un mode de réalisation, l'au moins un corps présente une dimension selon une hauteur de l'évidement comprise entre 2 mm et 7mm.

Selon un mode de réalisation, l'évidement comprend une première paroi de séparation configurée pour être positionnée entre l'au moins un corps et le dispositif de culture cellulaire, ladite première paroi de séparation comprenant au moins un orifice d'humidification positionné de sorte que lorsque le dispositif humidificateur est rempli du liquide humidificateur, l'au moins un orifice d'humidification est au-dessus d'une surface libre dudit liquide humidificateur.

La paroi latérale de l'évidement comprend la première paroi de séparation.

L'orifice d'humidification a pour but de permettre un transfert d'un gaz chargé de liquide humidificateur présent dans le dispositif humidificateur vers le dispositif de culture cellulaire de sorte à humidifier des puits de culture dudit dispositif de culture cellulaire.

Ainsi, l'orifice d'humidification doit présenter une dimension suffisamment importante pour permettre cet échange gazeux.

En outre, la première paroi de séparation a pour but de retenir le liquide humidificateur dans l'évidement. L'orifice humidificateur présente donc une dimension suffisamment petite pour former une barrière à un débordement du liquide humidificateur. Plus précisément, l'orifice humidificateur joue notamment sur la tension superficielle du liquide humidificateur pour empêcher le passage du liquide humidificateur par l'orifice humidificateur.

Selon un mode de réalisation, l'orifice humidificateur a une dimension inférieure à la dimension du canal selon la largeur de l'évidement.

Selon un mode de réalisation, l'orifice humidificateur a une dimension inférieure à 5mm.

Selon un mode de réalisation, la première paroi de séparation comprend une pluralité d'orifices humidificateurs.

Selon un mode de réalisation, un bord de l'orifice humidificateur est confondu avec un bord libre de la première paroi de séparation.

Selon un mode de réalisation, les orifices humidificateurs forment des créneaux sur le bord libre de la première paroi de séparation.

Selon un mode de réalisation, l'évidement comprend une deuxième paroi de séparation configurée pour être positionnée entre l'au moins un corps et un bord extérieur du dispositif de culture cellulaire, ladite deuxième paroi de séparation comprenant un orifice de débordement positionné de sorte que lorsque le dispositif humidificateur est rempli du liquide humidificateur, l'au moins un orifice de débordement est au-dessus d'une surface libre dudit liquide humidificateur.

La paroi latérale de l'évidement comprend la deuxième paroi de séparation.

L'orifice de débordement a pour but d'évacuer un excès de liquide humidificateur présent dans le dispositif humidificateur vers l'extérieur de celui-ci de sorte à éviter un débordement dans les puits de culture lors d'un mouvement du liquide humidificateur.

Ainsi, l'orifice de débordement doit présenter une dimension suffisamment importante pour permettre de facilement évacuer le liquide humidificateur.

Selon un mode de réalisation, la deuxième paroi de séparation comprend une pluralité d'orifices de débordement.

Selon un mode de réalisation, un bord de l'orifice de débordement est confondu avec un bord libre de la deuxième paroi de séparation.

Selon un mode de réalisation, l'au moins un orifice de débordement est positionné dans un angle du dispositif humidificateur.

En effet, lorsque le dispositif de culture subit un mouvement, il est probable que ledit mouvement entraine une inclinaison du dispositif de culture cellulaire favorisant un écoulement du liquide humidificateur vers un angle du dispositif humidificateur.

L'orifice de débordement est positionné suffisamment au-dessus de la surface libre du liquide humidificateur de sorte que le liquide humidificateur puisse s'accumuler dans l'angle du dispositif humidificateur, mais qu'une accumulation excessive soit évacuée sur le bord extérieur du dispositif de culture cellulaire par l'orifice de débordement.

L'invention porte également sur un kit comprenant au moins une boîte de culture munie d'un dispositif humidificateur et au moins une plaque de culture.

L'invention porte également sur un procédé de dimensionnement d'un dispositif humidificateur d'un gaz pour un dispositif de culture cellulaire comprenant :
- une étape de création d'au moins un évidement configuré pour recevoir un liquide humidificateur ;
- une étape de positionnement d'au moins un corps dans l'évidement de sorte que l'au moins un corps sépare une longueur dudit évidement afin de former un premier réservoir et un deuxième réservoir reliés l'un à l'autre par un canal ;
ledit canal permettant un passage du liquide humidificateur du premier réservoir vers le deuxième réservoir de sorte qu'une totalité de l'évidement est rempli en introduisant le liquide humidificateur dans un unique réservoir, le liquide humidificateur qui est versé dans le premier réservoir s'écoule vers le deuxième réservoir à travers le canal lorsque ledit premier réservoir est complétement rempli, et le liquide humidificateur placé dans l'évidement est maintenu dans celui-ci lorsque le dispositif humidificateur est soumis à des déplacements.

L'invention sera mieux comprise, grâce à la description ci-après, qui se rapporte à plusieurs modes de réalisation selon la présente invention, donné à titre d'exemples non limitatifs et expliqués avec référence aux dessins schématiques annexés, dans lesquels :
[FIG. 1] est une vue explosée d'une boîte de culture comprenant un dispositif humidificateur selon l'invention et au moins une plaque de culture;
[FIG. 2] est une vue de la boîte de culture dans laquelle l'au moins une plaque de culture est insérée dans un logement ;
[FIG. 3] est une vue de la boîte de culture dans laquelle l'au moins une plaque de culture est insérée dans un logement et dans laquelle une plaque de fond est fixée;
[FIG. 4] est une vue de la boîte de culture en position fermée ;
[FIG. 5] est une vue d'un élément intermédiaire comprenant le dispositif humidificateur selon l'invention vue de dessus ;
[FIG. 6] est une vue tridimensionnelle de l'élément intermédiaire et un détail de celui-ci ;
[FIG. 7] est une représentation en coupe du dispositif humidificateur selon l'invention ;
[FIG. 8] est une représentation de différents modes de réalisation du dispositif humidificateur ; [FIG. 9] est une vue d'un module complémentaire vue de côté ;
[FIG. 10] est une vue tridimensionnelle et en coupe de l'élément intermédiaire sous lequel le module complémentaire est positionné;
[FIG. 11] est une vue tridimensionnelle et en coupe de l'élément intermédiaire sous lequel le module complémentaire est positionné afin de permettre un retrait de la plaque de culture;
[FIG. 12] est une vue tridimensionnelle et en coupe de l'élément intermédiaire dans lequel le module complémentaire est positionnée dans un logement;

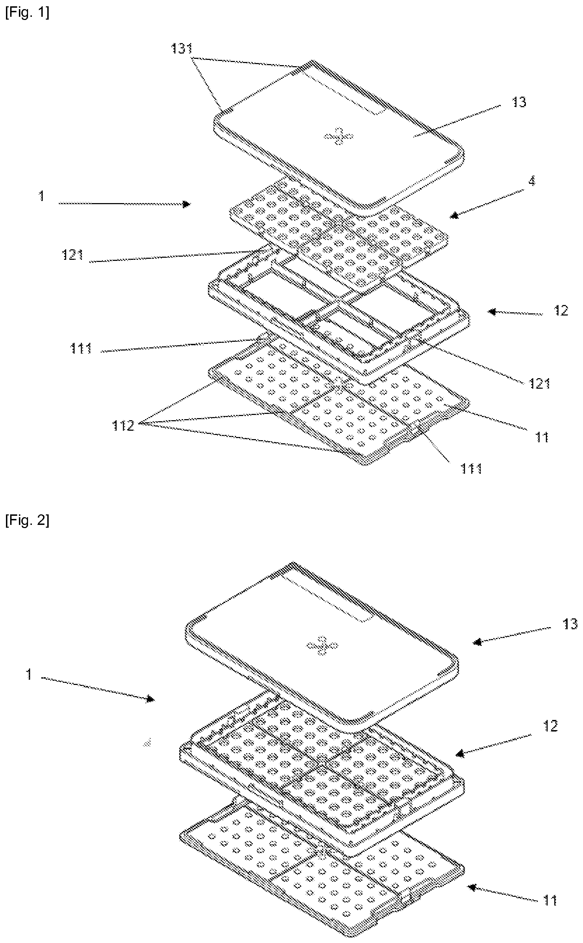

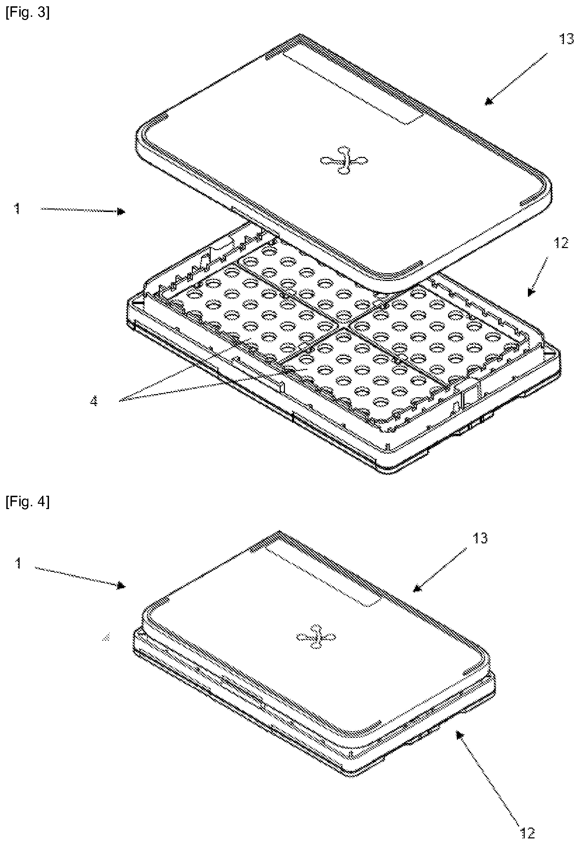

Un dispositif de culture cellulaire comme une boîte de culture 1 comprenant un dispositif humidificateur 122 selon l'invention est représentée sur les figures 1 à 4. Elle comprend une plaque de fond 11, un élément intermédiaire 12 et un couvercle 13.

La plaque de fond 11 s'étend sensiblement dans un plan. La plaque de fond 11 a une forme non symétrique, c'est-à-dire qu'elle a une forme sensiblement rectangulaire avec trois angles arrondis et un angle droit. Sur au moins un bord, elle comprend des premiers moyens d'attache 111. La plaque de fond 11 comprend également au moins un épaulement de centrage 112.

Le couvercle 13 a une forme sensiblement identique à une partie supérieure de l'élément intermédiaire 12, dans le cas présent, le couvercle 13 a une forme non symétrique, c'est-à-dire qu'il a une forme sensiblement rectangulaire avec trois angles arrondis et un angle droit, de sorte à pouvoir être emboîté sur l'élément intermédiaire 12. Le couvercle 13 comprend également au moins un épaulement de centrage 131 sur une face intérieure et/ou sur une face extérieure pour permettre un empilement de plusieurs boîtes de culture 1.

L'élément intermédiaire 12 a une forme sensiblement identique à la plaque de fond 11 dans une partie inférieure et au couvercle 13 dans la partie supérieure. Dans le cas présent, l'élément intermédiaire 12 a une forme non symétrique, c'est-à-dire qu'il a une forme sensiblement rectangulaire avec trois angles arrondis et un angle droit. Sur au moins un bord, il comprend des troisièmes moyens d'attache 121.

L'élément intermédiaire 12 comprend au moins un logement 2 permettant de positionner et de maintenir une plaque de culture 4 ou un module complémentaire 3.

Le logement 2 comprend une paroi de maintien 21 configurée pour venir en contact avec, et plus particulièrement à entourer, un bord de la plaque de culture 4. La paroi de maintien 21 entoure le logement 2.

La paroi de maintien 21 est configurée pour permettre l'introduction de la plaque de culture 4 dans le logement 2 en prenant en compte des tolérances de fabrication de la plaque de culture 4. La paroi de maintien 21 permet de positionner dans un plan de maintien la plaque de culture 4 lorsque cette dernière est insérée dans le logement 2. Préférentiellement, le plan de maintien est un plan dans lequel s'étend la boîte de culture 1.

Le logement 2 comprend également une surface d'appui 22 solidaire de la paroi de maintien 21, ladite surface d'appui 22 étant configurée pour venir partiellement en contact avec une première surface d'élongation de la plaque de culture 4. Autrement dit, lorsque la plaque de culture 4 est positionnée dans le logement 2, la première surface d'élongation est en contact avec la surface d'appui 22. Préférentiellement, la surface d'appui 22 permet de maintenir la plaque de culture 4 suivant une direction de maintien sensiblement perpendiculaire au plan de maintien.

Le logement 2 comprend en outre au moins un dispositif de fixation 23 de la plaque de culture 4. Chaque dispositif de fixation 23 est solidaire de la paroi de maintien 21.

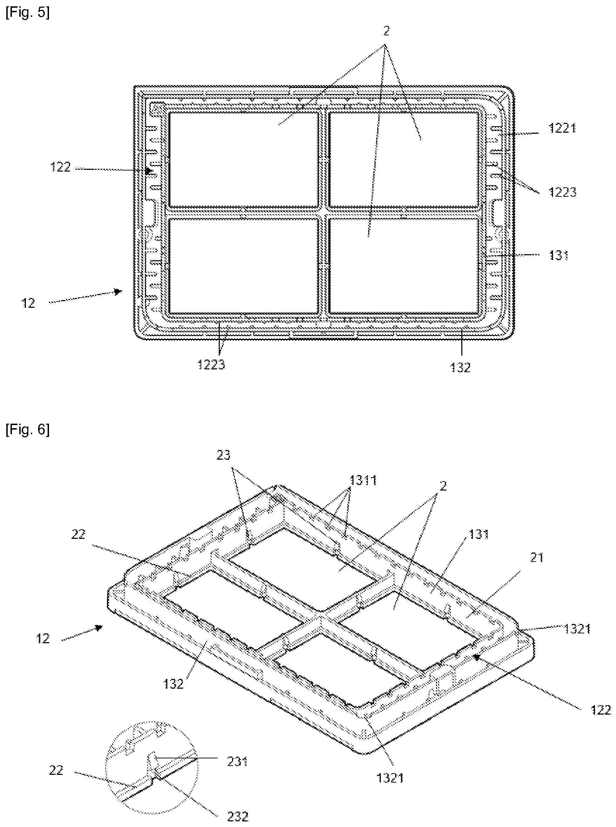

Le dispositif de fixation 23 comprend, comme illustrée en figure 6, une surface de retenue 232 positionnée en regard de la surface d'appui 22. La surface de retenue 232 comprend une première extrémité s'étendant à distance de la paroi de maintien 21 et une deuxième extrémité attachée directement ou par l'intermédiaire d'un élément de liaison à la paroi de maintien 21.

La surface de retenue 232 s'écarte de la paroi de maintien 21 lorsqu'elle s'éloigne de la surface d'appui 22. L'inclinaison de la surface de retenue 232 facilite un retrait de la plaque de culture 4 du logement 2. La surface de retenue 232 agit comme une surface d'éjection de la plaque de culture 4.

Un espace de maintien est délimité entre la surface de retenue 232 et la surface d'appui 22. L'espace de maintien a une dimension correspondant à au moins une épaisseur de la plaque de culture 4 destinée à venir en regard de l'au moins une surface de retenue 232.

L'espace de maintien présente une dimension permettant une insertion de la plaque de culture 4 entre la surface d'appui 22 et la surface de retenue 232. Autrement dit, la dimension de l'espace de maintien correspond au minimum à l'épaisseur de la plaque de culture 4 prise au niveau de la surface de retenue 232 lorsque la plaque de culture 4 est positionnée dans le logement 2. L'espace de maintien peut être augmenté d'un jeu fonctionnel de sorte à prendre en compte des tolérances de fabrication de la plaque de culture 4. Ainsi, lorsque la plaque de culture 4 est insérée dans l'espace de maintien aucune force mécanique ou contrainte pouvant entrainer une déformation de la plaque de culture 4 ne s'exerce sur cette dernière.

Selon un mode de réalisation, l'au moins un dispositif de fixation 23 comprend également au moins une surface d'introduction 231, comme illustrée en figure 6. La surface d'introduction 231 s'écarte de la paroi de maintien 21 lorsqu'elle s'approche de la surface d'appui 22. La surface d'introduction 231 facilite l'insertion de la plaque de culture 4 dans le logement 2 et plus particulièrement dans l'espace de maintien en formant une rampe d'insertion.

Le dispositif de fixation 23 permet un emboitage élastique de la plaque de culture 4 dans le logement 2. Ainsi, la plaque de culture 4 peut être insérée dans l'espace de maintien sans qu'une force mécanique ou contrainte pouvant entrainer une déformation de la plaque de culture 4 ne s'exerce sur cette dernière après insertion, c'est-à-dire une fois en place. Lorsque la plaque de culture 4 est introduite dans le logement 2 elle est parfaitement maintenue dans le plan de maintien et selon la direction de maintien de sorte que la plaque de culture 4 est maintenue dans toutes les directions de l'espace quelle que soit l'orientation de la boîte de culture 1.

L'élément intermédiaire 12 comprend également un dispositif humidificateur 122. Le dispositif humidificateur 122 comprend deux évidements qui entourent un bord de l'élément intermédiaire 12 de sorte qu'un liquide humidificateur L peut se répartir sur l'ensemble du bord de l'élément intermédiaire 12. Plus précisément, le dispositif humidificateur 122 est configuré pour être positionné sur un bord de l'élément intermédiaire 12 qui est un dispositif de culture cellulaire.

Plus précisément, le dispositif humidificateur 122 permet d'humidifier un gaz présent dans la boite de culture 1. Préférentiellement, le liquide humidificateur L comprend au moins 90% d'eau, préférence 99% d'eau, et par exemple 100% d'eau et le gaz entourant le boîte de culture contient de la vapeur d'eau et de l'oxygène, et est préférentiellement de l'air avec un taux d'humidité supérieur à 80%.

Le dispositif humidificateur 122 comprend au moins un évidement 1221 configuré pour recevoir le liquide humidificateur L. Dans les figures 5 à 8, le dispositif humidificateur 122 comprend deux évidements 1221.

Préférentiellement, l'évidement 1221 n'est pas fermé de sorte à laisser une surface libre au liquide humidificateur L et ainsi favoriser l'évaporation de ce dernier. L'évidement 1221 comprend donc au moins un fond 1222, et une paroi latérale 1224.

Le fond 1222 s'étend dans un plan parallèle à un plan d'élongation du dispositif humidificateur 122, correspondant au plan d'élongation de la boîte de culture 1.

La paroi latérale 1224 s'étend avec un angle par rapport au plan d'élongation du dispositif humidificateur 122. Selon un mode de réalisation, l'angle est compris entre [30°, 150°], par exemple [60°, 120°] et préférentiellement 90°.

Selon un mode de réalisation, une largeur y de l'évidement 1221 est comprise entre 2 et 10mm, de préférence entre 2 et 6mm, par exemple 5mm.

Selon un mode de réalisation, l'évidement 1221 est réalisé dans un matériaux choisi parmi : un acide polylactique, un polystyrène et un polydiméthylsiloxane ou diméthicole.

Ledit évidement 1221 est muni d'au moins un corps 1223, et dans le cas présent d'une pluralité de corps 1223, séparant une longueur dudit évidement de sorte à former un premier réservoir R1 et un deuxième réservoir R2 reliés l'un à l'autre par un canal R3.

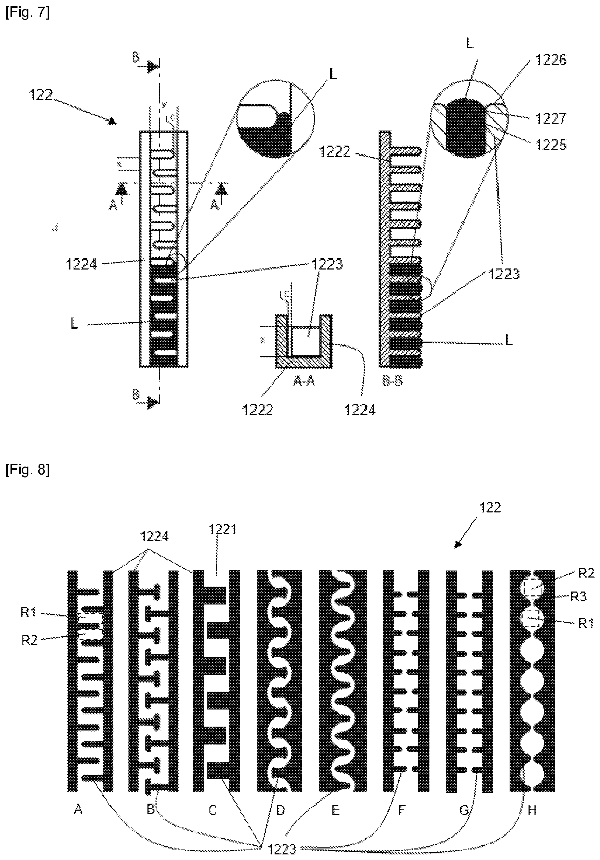

Dans les exemples illustrés en figures 5 et 8, l'évidement 1221 comprend une pluralité de corps 1223 positionnés régulièrement et/ou alternativement de part et d'autre d'un centre de l'évidement 1221.

On entend par des corps 1223 positionnés régulièrement, qu'une distance entre les corps 1223 pris selon un axe, comme par exemple la longueur de l'évidement 1221 est constante. C'est le cas de tous les exemples représentés.

On entend par des corps 1223 positionnés alternativement de part et d'autre d'un centre de l'évidement 1221, que deux corps 1223 successifs sont positionnés d'un côté et de l'autre côté du centre de l'évidement 1221, qui est par exemple pris selon la longueur de l'évidement 1221. C'est le cas des exemple de la figure 5 et des exemples A, B, C, D, E, et F de la figure 8.

Selon un mode de réalisation, les corps 1223 sont positionnés d'un même côté de l'évidement 1221 ou des deux côtés de l'évidement 1221 comme illustré aux exemples G et H de la figure 8.

Selon un mode de réalisation, l'évidement 1221 comprend une pluralité de corps 1223 définissant un chemin continu formant une sinuosité comme illustré aux exemple de la figure 5 et les exemples A, B, C, D, E de la figure 8.

Selon un mode de réalisation, le corps 1223 comprend une surface de freinage 1225 configurée pour être au contact du liquide humidificateur L, et une surface de retenue 1226 s'étendant dans un plan formant un angle avec un plan dans lequel s'étend la surface de freinage 1225, ledit angle étant compris dans une première gamme de valeur.

La surface de freinage 1225 du corps 1223 relie le fond 1222 de l'évidement 1221 à la surface de retenue 1226 du corps1223. La surface de freinage 1225 s'étend avec un angle par rapport au plan dans lequel s'étend le dispositif humidificateur 122, l'angle étant compris entre [20° ,160°], par exemple [60° ,120°], et préférentiellement entre [89°, 92°]. Dans ce dernier cas, la surface de freinage 1225 correspond à une hauteur z du corps 1223.

La surface de retenue 1226 s'étend dans un plan formant un angle compris dans une première gamme de valeur avec un plan dans lequel s'étend la surface de freinage 1225. La première gamme de valeur est comprise dans l'intervalle [20°, 160°], par exemple [60°, 120°], et de préférence [90°]. Dans ce dernier cas, la surface de retenue 1226 forme un angle droit avec la surface de freinage 1225.

Selon un mode de réalisation, lorsque la surface de freinage 1225 s'étend perpendiculairement par rapport au fond 1222 de l'évidement 1221, la surface de retenue 1226 s'étend perpendiculairement à la surface de freinage 1225. Autrement dit, la surface de retenue 1226 s'étend dans le plan d'élongation du dispositif humidificateur 122.

La surface de freinage 1225 est reliée à la surface de retenue 1226 par une surface courbe 1227 présentant un rayon de courbure inférieur à 0.5mm, par exemple inférieur à 0,2mm, et de préférence 0mm.

Le rayon de courbure de la surface courbe 1227 fait varier une tension superficielle du liquide humidificateur L au contact de la surface courbe 1227. Ainsi, la surface courbe 1227 permet de favoriser une retenue du liquide humidificateur L dans le réservoir R1, R2. La surface courbe 1227 diminue le mouvement du liquide humidificateur L selon un plan perpendiculaire au plan d'élongation du dispositif humidificateur 122. La géométrie du corps 1223 permet de diminuer le risque que le liquide humidificateur L sorte du réservoir R1, R2 pour inonder un puit de la plaque de culture 4.

Selon un mode de réalisation, l'au moins un corps 1223 présente une dimension selon une hauteur de l'évidement 1221 comprise entre 1mm et 15mm, par exemple entre 2mm et 7mm. Dans le cas d'un évidement 1221 comprenant une pluralité de corps, un réservoir R1, R2 est compris entre deux canaux R3 ou entre un canal R3 et la paroi latérale 1224 de l'évidement 1221. Ainsi, deux corps 1223 successifs définissent un réservoir en commun.

Le canal R3 permet un passage du liquide humidificateur L du premier réservoir R1 vers le deuxième réservoir R2 de sorte que l'ensemble de l'évidement 1221 peut être rempli en introduisant le liquide humidificateur L dans un unique réservoir. Autrement dit, le liquide humidificateur L s'écoule du premier réservoir R1 vers le deuxième réservoir R2 à travers le canal R3. Ainsi, un remplissage de l'évidement 1221 est rapide et facile car il peut être réalisé en versant le liquide humidificateur L dans un seul réservoir. Le canal R3 présente une taille suffisante par rapport aux propriétés physiques du liquide humidificateur, comme par exemple la tension superficielle, la viscosité cinématique, la densité, la mouillabilité du liquide humidificateur, pour permettre un écoulement du liquide humidificateur L du premier réservoir R1 vers le deuxième réservoir R2.

Selon un mode de réalisation, le liquide humidificateur L qui est versé dans le premier réservoir R1 s'écoule vers le deuxième réservoir R2 à travers le canal R3 lorsque ledit premier réservoir R1 est au moins en partie rempli. Autrement dit, le canal R3 par ces dimensions bloque le passage du liquide L tant que le premier réservoir R1 ne présente pas un certain niveau de remplissage. Par exemple, le canal peut bloquer le passage du liquide tant que le premier réservoir n'est pas rempli au moins au 3/4. Ainsi, le dispositif humidificateur 122 est rempli réservoir par réservoir.

En outre, le corps 1223 est positionné dans l'évidement 1221 suivant les critères ci-dessous :
- (1) une dimension c du canal R3 selon une largeur y de l'évidement 1221 est inférieure ou égale à la moitié de la largeur y de l'évidement 1221;
- (2) une dimension x d'au moins un des réservoirs R1, R2 selon la longueur de l'évidement 1221 est inférieure ou égale à la largeur y de l'évidement 1221;
- (3) la dimension x du réservoir R1, R2 selon la longueur de l'évidement 1221 est supérieure ou égale à la dimension c du canal R3 selon la largeur y de l'évidement 1221.

Le critère (1) impose que le canal R3 forme une constriction de l'évidement 1221. Ainsi, le canal R3 augmente des pertes de charges du mouvement du liquide humidificateur L et donc ralenti ce mouvement. Le canal R3 permet de former des réservoirs d'un volume inférieur à celui de l'évidement 1221 tout en conservant l'avantage d'un grand volume total de l'évidement 1221 qui est facilement rempli via un seul réservoir.

Le critère (2) limite la quantité de liquide humidificateur L présent dans chaque réservoir R1, R2 en limitant le volume dudit réservoir. En effet, les dimensions du réservoir R1, R2 selon la largeur y et la longueur de l'évidement 1221 est au plus égale à la largeur y de l'évidement 1221. Ainsi, le volume maximum du réservoir R1, R2, et donc le volume de liquide humidificateur L, est limité par une surface carrée de côté égale à la largeur y de l'évidement 1221.

Limiter le volume du liquide humidificateur L permet d'une part de limiter la masse de liquide humidificateur déplacée dans chaque réservoir lors d'un ballotements, et d'autre part d'augmenter un rapport entre un niveau de frottement entre le liquide humidificateur L et la paroi latérale 1224 de l'évidement 1221, et le volume du liquide humidificateur L. Ainsi, il faut plus d'énergie pour mettre en mouvement le liquide humidificateur L dans le réservoir R1, R2 que dans l'évidement 1221 sans corps 1223, et le liquide humidificateur acquière moins d'énergie cinétique dans un volume restreint, ce qui diminue les mouvements du liquide humidificateur L.

Enfin, le critère (3) impose un volume minimum du réservoir R1, R2. En effet, une dimension du réservoir R1, R2 selon la longueur de l'évidement est supérieure ou égale à la dimension c du canal R3 selon la largeur y de l'évidement 1221. Un volume minimum du réservoir est nécessaire afin qu'il ne constitue pas en-lui-même un canal et qu'il permette d'assurer une bonne humidification du dispositif de culture cellulaire.

Selon un mode de réalisation, la dimension c du canal R3 selon la largeur y de l'évidement 1221 est comprise entre 0,5mm et 5mm, et de préférence entre 0,9mm et 2,9mm.

Enfin, l'évidement 1221 et le corps 1223 du dispositif humidificateur 122 sont dimensionnés de sorte que le liquide humidificateur L placé dans l'évidement 1221 soit retenu dans celui-ci lorsque le dispositif humidificateur 122 est soumis à des déplacements, c'est-à-dire des vibrations.

Par ailleurs, selon un mode de réalisation, l'évidement 1221 comprend une première paroi de séparation 131 configurée pour être positionnée entre l'au moins un corps 1223 et le dispositif de culture cellulaire 4, ladite première paroi de séparation 131 comprenant au moins un orifice d'humidification 1311 positionné de sorte que lorsque le dispositif humidificateur 122 est rempli du liquide humidificateur L, l'au moins un orifice d'humidification 1311 est au-dessus d'une surface libre dudit liquide humidificateur L.

La paroi latérale 1224 de l'évidement 1221 comprend la première paroi de séparation 131.

L'orifice d'humidification 1311 a pour but de permettre un transfert d'un gaz chargé de liquide humidificateur L présent dans le dispositif humidificateur 122 vers le dispositif de culture cellulaire 4 de sorte à humidifier des puits de culture dudit dispositif de culture cellulaire.

Ainsi, l'orifice d'humidification 122 doit présenter une dimension suffisamment importante pour permettre cet échange gazeux.

En outre, la première paroi de séparation 131 a pour but de retenir le liquide humidificateur L dans l'évidement 1221. L'orifice humidificateur 1311 présente donc une dimension suffisamment petite pour former une barrière à un débordement du liquide humidificateur L. Plus précisément, l'orifice humidificateur 1311 joue notamment sur la tension superficielle du liquide humidificateur L pour empêcher le passage du liquide humidificateur L par l'orifice humidificateur 1311.

Selon un mode de réalisation, l'orifice humidificateur 1311 a une dimension inférieure à la dimension c du canal R3 selon la largeur y de l'évidement 1221.Selon un mode de réalisation, l'orifice humidificateur 1311 a une dimension inférieure à 5mm.

Selon un mode de réalisation, la première paroi de séparation 131 comprend une pluralité d'orifices humidificateurs 1311.

Selon un mode de réalisation, un bord de l'orifice humidificateur 1311 est confondu avec un bord libre de la première paroi de séparation 131.

Selon un mode de réalisation, les orifices humidificateurs 1311 forment des créneaux sur le bord libre de la première paroi de séparation 131 comme sur les figures.

Selon un mode de réalisation, l'évidement 1221 comprend une deuxième paroi de séparation 132 configurée pour être positionnée entre l'au moins un corps 1223 et un bord extérieur du dispositif de culture cellulaire 12, ladite deuxième paroi de séparation 132 comprenant un orifice de débordement 1321 positionné de sorte que lorsque le dispositif humidificateur 122 est rempli du liquide humidificateur L, l'au moins un orifice de débordement 1321 est au-dessus d'une surface libre dudit liquide humidificateur L.

La paroi latérale 1224 de l'évidement 1221 comprend la deuxième paroi de séparation 132.

L'orifice de débordement 1321 a pour but d'évacuer un excès de liquide humidificateur L présent dans le dispositif humidificateur 122 vers l'extérieur de celui-ci de sorte à éviter un débordement dans les puits de culture lors d'un mouvement du liquide humidificateur L.

Ainsi, l'orifice de débordement 1321 doit présenter une dimension suffisamment importante pour permettre de facilement évacuer le liquide humidificateur L.

Selon un mode de réalisation, la deuxième paroi de séparation 132 comprend une pluralité d'orifices de débordement 1321.

Selon un mode de réalisation, un bord de l'orifice de débordement 1321 est confondu avec un bord libre de la deuxième paroi de séparation 132.

Selon un mode de réalisation, l'au moins un orifice de débordement 1321 est positionné dans un angle du dispositif humidificateur 122.

En effet, lorsque le dispositif de culture 1 subit un mouvement, il est probable que ledit mouvement entraine une inclinaison du dispositif de culture cellulaire 1 favorisant un écoulement du liquide humidificateur L vers un angle du dispositif humidificateur 122.

L'invention porte également sur un procédé de dimensionnement d'un dispositif humidificateur 122 d'un gaz pour un dispositif de culture cellulaire comprenant :
- une étape de création d'au moins un évidement 1221 configuré pour recevoir un liquide humidificateur L ;
- une étape de positionnement d'au moins un corps 1223 dans l'évidement 1221 de sorte que l'au moins un corps 1223 sépare une longueur dudit évidement 1221 afin de former un premier réservoir R1 et un deuxième réservoir R2 reliés l'un à l'autre par un canal R3 ;
ledit canal R3 permettant un passage du liquide humidificateur L du premier réservoir R1 vers le deuxième réservoir R2 de sorte qu'une totalité de l'évidement 1221 est rempli en introduisant le liquide humidificateur L dans un unique réservoir, le liquide humidificateur L qui est versé dans le premier réservoir R1 s'écoule vers le deuxième réservoir R2 à travers le canal R3 lorsque ledit premier réservoir R1 est complétement rempli, et le liquide humidificateur L placé dans l'évidement 1221 est maintenu dans celui-ci lorsque le dispositif humidificateur 122 est soumis à des déplacements.

Par exemple, lorsque l'évidement 1221 est réalisé en acide polylactique et que le liquide humidificateur L est de l'eau, les corps 1223 de l'évidement 1221 peuvent être dimensionnés de la manière suivante :
- la dimension x des réservoirs R1, R2 selon la longueur de l'évidement 1221 : 2,9mm
- la largeur y de l'évidement 1221 : 5,3mm
- la hauteur z du corps 1223 : 5,8mm
- la dimension c du canal R3 selon la largeur y de l'évidement 1221 : 1,4mm

Un autre exemple donne que lorsque l'évidement 1221 est réalisé en polystyrène et que le liquide humidificateur L est de l'eau, les corps 1223 de l'évidement 1221 peuvent être dimensionnés de la manière suivante :
- la dimension x des réservoirs R1, R2 selon la longueur de l'évidement 1221 : 3,0mm
- la largeur y de l'évidement 1221 : 5,3mm
- la hauteur z du corps 1223 : 5,8mm
- la dimension c du canal R3 selon la largeur y de l'évidement 1221 : 1,7mm

La plaque de fond 11, le couvercle 13 et l'élément intermédiaire 12 sont configurés pour être emboîtée ensemble de manière séparables. Plus précisément, et comme illustré en figures 1 à 4, les premiers moyens d'attache 111 de la plaque de fond 11 coopèrent avec les troisièmes moyens d'attache 121 de l'élément intermédiaire 12, tandis que le couvercle 13 est emboîté sans maintien spécifique sur l'élément intermédiaire 12.. Ainsi, la plaque de fond 11 et le couvercle 13 peuvent être assemblés sur l'élément intermédiaire 12.

En outre, l'au moins un épaulement de centrage 112 de la plaque de fond 11 permet de positionner facilement l'élément intermédiaire 12 sur cette dernière afin de réaliser facilement la fixation de l'élément intermédiaire 12 sur la plaque de fond 11.

L'au moins un épaulement de centrage de la face extérieure 131 du couvercle 13 permet d'empiler facilement plusieurs boîtes de culture 1 l'une au-dessus de l'autre. Plus précisément, l'épaulement de centrage 131 peut coopérer avec la plaque de fond 11 ou avec l'élément intermédiaire 12. Ainsi, il est possible d'empiler plusieurs boîtes de culture comprenant la plaque de fond 11, ou de manière alternative sans la plaque de fond 11.

La boîte de culture 1 peut être utilisée avec ou sans la plaque de fond 11. La plaque de fond 11 permet de protéger, notamment lors d'une manipulation, l'au moins une plaque de culture 4 positionnée dans le logement 2 de l'élément intermédiaire 12. En outre, la plaque de fond 11 présente des caractéristiques d'épaisseur et de transparence permettant une imagerie microscope à moyen grossissement, par exemple x10, x20 ..., du contenu de la plaque de culture 4. La plaque de fond 11 peut être retirée notamment pour réaliser une imagerie microscope à fort grossissement, ou pour réaliser une imagerie confocale du contenu de la plaque de culture 4, ou encore pour une manipulation de la boîte de culture 1 par des automates de laboratoire.

Le logement 2 de la boîte de culture 1 est destiné à positionner et maintenir une plaque de culture 4 comme par exemple une plaque de culture 4 pour une application microfluidique.

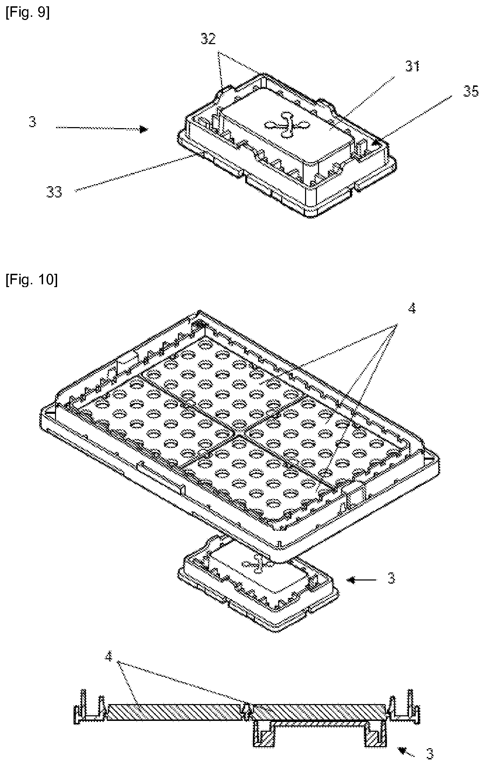

En outre, la boîte de culture 1 selon l'invention peut coopérer avec un module complémentaire 3 illustré aux figures 9 à 12.

Selon un mode de réalisation, le module complémentaire 3 est physiquement indépendant de la boîte de culture 1. Autrement dit, il n'y a pas de lien physique entre la boîte de culture 1 et le module complémentaire 3 qui peut donc être éloigné de la boîte de culture 1 d'une distance non prédéterminée. Plus précisément, il peut être positionné de chaque côté de l'élément intermédiaire 12.

Selon un mode de réalisation, le module complémentaire 3 a une forme qui permet son insertion dans le logement 2 de la boîte de culture 1.

Selon un mode de réalisation, le module complémentaire 3 a une forme symétrique.

Selon un mode de réalisation, le module complémentaire a une forme non symétrique, par exemple il a une forme sensiblement rectangulaire avec trois angles arrondis et un angle droit.

La forme permet de réaliser un détrompeur permettant de facilement orienter ledit module complémentaire 3.

Selon un mode de réalisation, le module complémentaire 3 à une forme identique à celle de la plaque de culture 4.

Le module complémentaire 3 permet notamment de retirer facilement la plaque de culture 4 du logement 2. Pour cela, le module complémentaire 3 comprend une surface de retrait 31 configurée pour venir partiellement en contact avec la première surface d'élongation de la plaque de culture 4. La surface de retrait 31 a pour objectif de soutenir la plaque de culture 4 lors de son retrait du logement 2 en évitant une déformation de cette dernière. La surface de retrait 31 vient donc au contact de la première surface d'élongation, et plus particulièrement d'une zone centrale de la première surface d'élongation.

Selon un mode de réalisation, la surface de retrait 31 s'étend sensiblement dans un plan de retrait.

Le module complémentaire 3 comprend également au moins un élément de retrait 32 configuré pour être positionné en regard d'un dispositif de fixation 23, et pour exercer une force localisée sur la plaque de culture 4 au niveau du dispositif de fixation 23.

Selon un mode de réalisation, le module complémentaire 3 comprend autant d'élément de retrait 32 que le logement 2 comprend de dispositif de fixation 23.

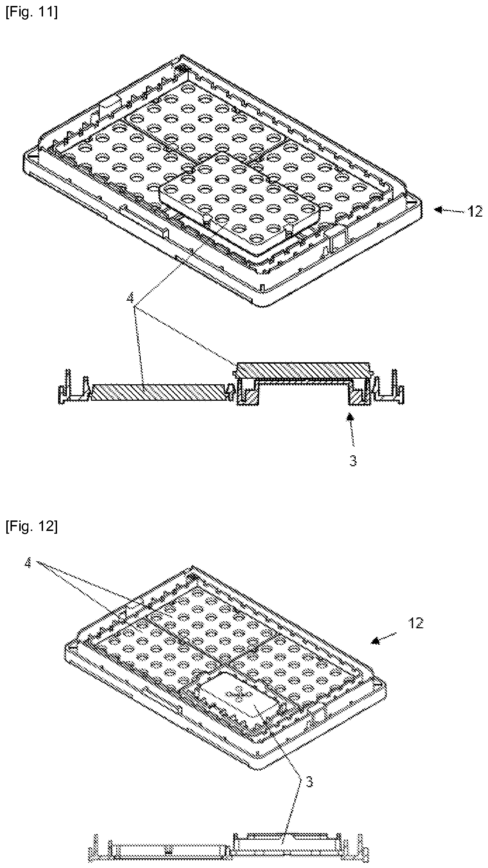

Pour retirer une plaque de culture 4 du logement 2, le module complémentaire 3 est positionné en regard de la première surface d'élongation de la plaque de culture 4 comme illustré en figure 10. Puis on amène le module complémentaire 3 en contact de ladite première surface d'élongation comme illustré en figure 11. La plaque de culture 4 est alors positionnée sur la surface de retrait 31 et sur les éléments de retrait 32 du module complémentaire 3. La plaque de culture 4 est alors extraite du logement 2 et peut être ôtée du module complémentaire 3.

L'élément de retrait 32 permet d'exercer une force localisée sur la plaque de culture 4 de sorte à réaliser une déformation locale de la plaque de culture 4, ou, par l'intermédiaire de la plaque de culture 4, une déformation du dispositif de fixation 23, afin de retirer la plaque de culture 4 de la boîte de culture 1 sans déformer cette dernière de manière globale.

Selon un mode de réalisation, la surface de retrait 31 et l'élément de retrait 32 s'étendent sensiblement dans un même plan. Ainsi, la surface de retrait 31 et l'élément de retrait 32 constitue un support plan à la plaque de culture 4 qui peut être souple, ce qui limite la propension de la plaque de culture 4 à emmagasiner de l'énergie en flexion. En effet, la plaque de culture 4 a tendance à restituer cette énergie en à-coup ou soubresaut, faisant gicler ou se déverser le contenu des puits 42 de culture.

Selon un mode de réalisation, le module complémentaire 3 peut être inséré dans le logement 2 à la place d'une plaque de culture 4 comme illustré en figure 12. Pour cela, le module complémentaire 3 comprend au moins une surface de support 33 configurée pour venir en appui sur la surface d'appui 22. Selon un mode de réalisation, la surface de support 33 du module complémentaire 3 est sensiblement parallèle à la surface de retrait 31.

Selon un mode de réalisation, la surface de support 33 est espacée de la surface de retrait 31 par une épaisseur constante.

Selon un mode de réalisation, le module complémentaire 3 comprend au moins un bord, configuré pour coopérer avec l'au moins un dispositif de fixation 23 de la boîte de culture 1. Ainsi le module complémentaire 3 peut être inséré dans l'espace de maintien du dispositif de fixation 23.

Selon un mode de réalisation, le module complémentaire 3 comprend au moins un moyen fonctionnel parmi : un dispositif humidificateur 35, un moyen lumineux, un moyen de ventilation, un moyen de mesure chimique, un moyen de mesure d'acidité, un moyen de mesure d'humidité, un moyen de chauffage, un moyen d'ajouter ou de retirer au moins un fluide, un moyen de mesure biologique, un moyen de mesure d'une déformation mécanique d'au moins une plaque de culture 4 contiguë audit moyen de mesure de la déformation mécanique, un moyen de simulation.

Ainsi lorsque le module complémentaire 3 est positionné dans la boîte de culture 1, il amène un moyen fonctionnel supplémentaire.

Le dispositif humidificateur 35 peut être identique à celui de l'élément intermédiaire 12.

Notamment, le dispositif humidificateur 35 entoure de manière continue un bord du module complémentaire 3 de sorte qu'un liquide peut se répartir sur l'ensemble du bord du module complémentaire 3 et il comprend un évidement muni de corps permettant notamment d'amortir et de contrôler un mouvement du fluide. Le module complémentaire 3 positionne le moyen humidificateur 35 au plus proche des puits adjacents des plaques de culture 4, de façon à limiter une distance entre les puits et la source d'humidification.

## Revendications

1. Dispositif humidificateur (122) d'un gaz pour un dispositif de culture cellulaire (1), ledit dispositif humidificateur (122) comprenant au moins un évidement (1221) configuré pour recevoir un liquide humidificateur (L), ledit évidement (1221) étant muni d'au moins un corps (1223) séparant une longueur dudit évidement (1221) de sorte à former un premier réservoir (R1) et un deuxième réservoir (R2) reliés l'un à l'autre par un canal (R3), **caractérisé en ce que** l'au moins un corps (1223) est positionné dans l'évidement (1221) de sorte que :
- Une dimension (c) du canal (R3) selon une largeur (y) de l'évidement (1221) est inférieure ou égale à la moitié de la largeur (y) de l'évidement (1221);
- Une dimension (x) d'au moins un des réservoirs (R1, R2) selon la longueur de l'évidement (1221) est inférieure ou égale à la largeur (y) de l'évidement (1221);
- La dimension (x) du réservoir (R1, R2) selon la longueur de l'évidement (1221) est supérieure ou égale à la dimension (c) du canal (R3) selon une largeur (y) de l'évidement (1221).

2. Dispositif humidificateur (122) selon la revendication 1, dans lequel la largeur (y) de l'évidement (1221) est comprise entre 2 et 10mm, de préférence entre 2 et 6mm, par exemple 5mm.

3. Dispositif humidificateur (122) selon la revendication 1 ou 2, dans lequel la dimension du canal (c) selon la largeur (y) de l'évidement (1221) est comprise entre 0,5mm et 5mm, et de préférence entre 0,9mm et 2,9mm.

4. Dispositif humidificateur (122) selon l'une quelconque des revendications précédentes, dans lequel l'évidement (1221) est réalisé dans un matériaux choisi parmi : un acide polylactique, un polystyrène et un polydiméthylsiloxane ou diméthicole.

5. Dispositif humidificateur (122) selon l'une quelconque des revendications précédentes, dans lequel l'évidement (1221) comprend une pluralité de corps (1223) positionnés régulièrement et/ou alternativement de part et d'autre d'un centre de l'évidement (1221).

6. Dispositif humidificateur (122) selon l'une quelconque des revendications précédentes, dans lequel l'évidement (1221) comprend une pluralité de corps (1223) définissant un chemin continu formant une sinuosité.

7. Dispositif humidificateur (122) selon l'une quelconque des revendications précédentes, dans lequel le corps (1223) comprend une surface de freinage (1225) configurée pour être au contact du liquide humidificateur (L), et une surface de retenue (1226) s'étendant dans un plan formant un angle avec un plan dans lequel s'étend la surface de freinage (1225), ledit angle étant compris dans une première gamme de valeur, la surface de freinage (1225) étant reliée à la surface de retenue (1226) par une surface courbe (1227) présentant un rayon de courbure inférieur ou égale à 0.5mm.

8. Dispositif humidificateur (122) selon l'une quelconque des revendications précédentes, dans lequel l'au moins un corps (1223) présente une dimension selon une hauteur (z) de l'évidement comprise entre 1mm et 15mm.

9. Dispositif humidificateur (122) selon l'une quelconque des revendications précédentes, dans lequel l'évidement (1221) comprend une première paroi de séparation (131) configurée pour être positionnée entre l'au moins un corps (1223) et le dispositif de culture cellulaire (1), ladite première paroi de séparation (131) comprenant au moins un orifice d'humidification (1311) positionné de sorte que lorsque le dispositif humidificateur (122) est rempli du liquide humidificateur (L), l'au moins un orifice d'humidification (1311) est au-dessus d'une surface libre dudit liquide humidificateur (L).

10. Dispositif humidificateur (122) selon l'une quelconque des revendications précédentes, dans lequel l'évidement (1221) comprend une deuxième paroi de séparation (132) configurée pour être positionnée entre l'au moins un corps (1223) et un bord extérieur du dispositif de culture cellulaire (1), ladite deuxième paroi de séparation (132) comprenant un orifice de débordement (1321) positionné de sorte que lorsque le dispositif humidificateur (122) est rempli du liquide humidificateur (L), l'au moins un orifice de débordement (1321) est au-dessus d'une surface libre dudit liquide humidificateur (L).

11. Kit comprenant au moins une boîte de culture (1) munie d'un dispositif humidificateur (122) selon l'une quelconque des revendications précédentes et au moins une plaque de culture (4).

## Patentansprüche

1. Gasbefeuchtungsvorrichtung (122) für eine Zellkulturvorrichtung (1), wobei die Befeuchtungsvorrichtung (122) mindestens eine Aussparung (1221) umfasst, die zur Aufnahme einer Befeuchtungsflüssigkeit (L) eingerichtet ist, wobei die Aussparung (1221) mit mindestens einem Körper (1223) versehen ist, der eine Länge von der Aussparung (1221) trennt, um einen ersten Behälter (R1) und einen zweiten Behälter (R2) zu bilden, die durch einen Kanal (R3) miteinander verbunden sind, **dadurch gekennzeichnet, dass** der mindestens eine Körper (1223) in der Aussparung (1221) so positioniert ist, dass:
- eine Abmessung (c) des Kanals (R3) entlang einer Breite (y) der Aussparung (1221) kleiner oder gleich der Hälfte der Breite (y) der Aussparung (1221) ist;
- eine Abmessung (x) mindestens eines der Behälter (R1, R2) je nach Länge der Aussparung (1221) kleiner oder gleich der Breite (y) der Aussparung (1221) ist;
- die Abmessung (x) des Behälters (R1, R2) entsprechend der Länge der Aussparung (1221) größer oder gleich dem Maß (c) des Kanals (R3) entsprechend einer Breite (y) der Aussparung (1221) ist.

2. Befeuchtungsvorrichtung (122) nach Anspruch 1, wobei die Breite (y) der Aussparung (1221) zwischen 2 und 10 mm, vorzugsweise zwischen 2 und 6 mm, beispielsweise 5 mm, beträgt.

3. Befeuchtungsvorrichtung (122) nach Anspruch 1 oder 2, wobei die Abmessung des Kanals (c) nach der Breite (y) der Aussparung (1221) zwischen 0,5 mm und 5 mm und vorzugsweise zwischen 0,9 mm und 2,9 mm beträgt.

4. Befeuchtungsvorrichtung (122) nach einem der vorhergehenden Ansprüche, wobei die Aussparung (1221) aus einem Material hergestellt ist, das ausgewählt ist aus: Polymilchsäure, Polystyrol und Polydimethylsiloxan oder Dimethicol.

5. Befeuchtungsvorrichtung (122) nach einem der vorhergehenden Ansprüche, wobei die Aussparung (1221) eine Vielzahl von Körpern (1223) umfasst, die regelmäßig und/oder abwechselnd auf beiden Seiten einer Mitte der Aussparung (1221) positioniert sind.

6. Befeuchtungsvorrichtung (122) nach einem der vorhergehenden Ansprüche, wobei die Aussparung (1221) eine Vielzahl von Körpern (1223) umfasst, die einen durchgehenden Weg definieren, der eine Windung bildet.

7. Befeuchtungsvorrichtung (122) nach einem der vorhergehenden Ansprüche, wobei der Körper (1223) eine Bremsfläche (1225) umfasst, die so eingerichtet ist, dass sie mit der Befeuchtungsflüssigkeit (L) in Kontakt steht, und eine Haltefläche (1226), die sich in einer Ebene erstreckt, die einen Winkel mit einer Ebene bildet, in der sich die Bremsfläche (1225) erstreckt, wobei der Winkel in einem ersten Wertebereich liegt, wobei die Bremsfläche (1225) mit der Haltefläche (1226) durch eine gekrümmte Fläche (1227) verbunden ist, die einen Krümmungsradius kleiner oder gleich 0,5 mm aufweist.

8. Befeuchtungsvorrichtung (122) nach einem der vorhergehenden Ansprüche, wobei der mindestens eine Körper (1223) eine Abmessung nach einer Höhe (z) der Aussparung zwischen 1 mm und 15 mm aufweist.

9. Befeuchtungsvorrichtung (122) nach einem der vorhergehenden Ansprüche, wobei die Aussparung (1221) eine erste Trennwand (131) umfasst, die so eingerichtet ist, dass sie zwischen dem mindestens einen Körper (1223) und der Zellkulturvorrichtung (1) positioniert ist, wobei die erste Trennwand (131) mindestens eine Befeuchtungsöffnung (1311) umfasst, die so positioniert ist, dass, wenn die Befeuchtungsvorrichtung (122) mit der Befeuchtungsflüssigkeit (L) gefüllt ist, die mindestens eine Befeuchtungsöffnung (1311) über einer freien Oberfläche der Befeuchtungsflüssigkeit (L) liegt.

10. Befeuchtungsvorrichtung (122) nach einem der vorhergehenden Ansprüche, wobei die Aussparung (1221) eine zweite Trennwand (132) umfasst, die so eingerichtet ist, dass sie zwischen dem mindestens einen Körper (1223) und einer Außenkante der Zellkulturvorrichtung (1) positioniert ist, wobei die zweite Trennwand (132) eine Überlauföffnung (1321) umfasst, die so positioniert ist, dass, wenn die Befeuchtungsvorrichtung (122) mit der Befeuchtungsflüssigkeit (L) gefüllt ist, die mindestens eine Überlauföffnung (1321) über einer freien Oberfläche der Befeuchtungsflüssigkeit (L) liegt.

11. Set, umfassend mindestens eine Kulturschale (1), die mit einer Befeuchtungsvorrichtung (122) nach einem der vorstehenden Ansprüche und mindestens einer Kulturscheibe (4) versehen ist.

## Claims

1. A device (122) for humidifying a gas for a cell culture device (1), said humidifying device (122) comprising at least one recess (1221) configured to receive a humidifying liquid (L), said recess (1221) being provided with at least one body (1223) separating a length of said recess (1221) so as to form a first reservoir (R1) and a second reservoir (R2) connected to each other by a channel (R3), **characterized in that** the at least one body (1223) is positioned in the recess (1221) such that:
- a dimension (c) of the channel (R3) along a width (y) of the recess (1221) is less than or equal to half the width (y) of the recess (1221);
- a dimension (x) of at least one of the reservoirs (R1, R2) along the length of the recess (1221) is less than or equal to the width (y) of the recess (1221);
- the dimension (x) of the reservoir (R1, R2) along the length of the recess (1221) is greater than or equal to the dimension (c) of the channel (R3) along a width (y) of the recess (1221).

2. The humidifying device (122) according to claim 1, wherein the width (y) of the recess (1221) is comprised between 2 and 10 mm, preferably between 2 and 6 mm, for example 5 mm.

3. The humidifying device (122) according to claim 1 or 2, wherein the dimension of the channel (c) along the width (y) of the recess (1221) is comprised between 0.5 mm and 5 mm, and preferably between 0.9 mm and 2.9 mm.

4. The humidifying device (122) according to any one of the preceding claims, wherein the recess (1221) is made of a material selected from: polylactic acid, polystyrene, and polydimethylsiloxane or dimethicone.

5. The humidifying device (122) according to any one of the preceding claims, wherein the recess (1221) comprises a plurality of bodies (1223) positioned regularly and/or alternately on either side of a center of the recess (1221).

6. The humidifying device (122) according to any one of the preceding claims, wherein the recess (1221) comprises a plurality of bodies (1223) defining a continuous path forming a sinuosity.

7. The humidifying device (122) according to any one of the preceding claims, wherein the body (1223) comprises a braking surface (1225) configured to be in contact with the humidifying liquid (L), and a retaining surface (1226) extending in a plane forming an angle with a plane in which the braking surface (1225) extends, said angle being comprised within a first range of values, the braking surface (1225) being connected to the retaining surface (1226) by a curved surface (1227) having a radius of curvature less than or equal to 0.5 mm.

8. The humidifying device (122) according to any one of the preceding claims, wherein the at least one body (1223) has a dimension along a height (z) of the recess comprised between 1 mm and 15 mm.

9. The humidifying device (122) according to any one of the preceding claims, wherein the recess (1221) comprises a first partition wall (131) configured to be positioned between the at least one body (1223) and the cell culture device (1), said first partition wall (131) comprising at least one humidification orifice (1311) positioned such that, when the humidifying device (122) is filled with the humidifying liquid (L), the at least one humidification orifice (1311) is above a free surface of said humidifying liquid (L).

10. The humidifying device (122) according to any one of the preceding claims, wherein the recess (1221) comprises a second partition wall (132) configured to be positioned between the at least one body (1223) and an outer edge of the cell culture device (1), said second partition wall (132) comprising an overflow orifice (1321) positioned such that, when the humidifying device (122) is filled with the humidifying liquid (L), the at least one overflow orifice (1321) is above a free surface of said humidifying liquid (L).

11. A kit comprising at least one culture dish (1) provided with a humidifying device (122) according to any one of the preceding claims and at least one culture plate (4).
